# EUROPÄISCHE PATENTSCHRIFT

(11) **EP 2 709 448 B1**
(45) Veröffentlichungstag und Bekanntmachung des Hinweises auf die Patenterteilung: **25.10.2017**
(21) Anmeldenummer: 12721508.5
(22) Anmeldetag: 11.05.2012
(51) Int. Cl.: A01N 25/10, A01N 25/34, A01N 59/16, A01P 1/00, B05B 11/00, A01N 59/20

(54) **VORRATSGEFÄSS ENTHALTEND EINEN ANTIBAKTERIELL WIRKENDEN FORMKÖRPER, VERFAHREN ZUR STERILISIERUNG VON FORMULIERUNGEN, SOWIE VERWENDUNG DES VORRATSGEFÄSSES**
STORAGE VESSEL COMPRISING ANTIBACTERIAL SHAPED FORM, METHOD FOR THE STERILISATION OF FORMULATIONS, AND USE OF THE STORAGE VESSEL
RÉSERVOIR DE STOCKAGE COMPRENANT UN CORPS MOULÉS ANTIBACTERIENS, UNE MÉTHODE POUR LA STERILISATION DES FORMULES, ET L'UTILSATION DU RÉSERVOIR DE STOCKAGE

(30) Priorität: 18.05.2011 DE 102011106303
(43) Veröffentlichungstag der Anmeldung: 26.03.2014
(73) Patentinhaber: F. Holzer GmbH, 66386 St. Ingbert (DE)
(72) Erfinder: LEE, Hyeck-Hee, 66386 St. Ingbert (DE); HOLZER, Frank, 66386 St. Ingbert (DE); STEINFELD, Ute, 66386 St. Ingbert (DE); MAHLER, Markus, 66333 Völklingen (DE)
(74) Vertreter: Pfenning, Meinig & Partner mbB
(86) Internationale Anmeldenummer: PCT/EP2012/058803
(87) Internationale Veröffentlichungsnummer: WO 2012/156321

(56) Entgegenhaltungen:
- EP-A2- 0 360 962
- EP-A2- 0 473 892
- EP-A2- 0 473 982
- WO-A1-00/37179
- WO-A1-2009/109370
- DE-A1- 19 605 153
- US-A1- 2003 118 658
- US-A1- 2009 060 967
- "Sterile vials containing a solution of docetaxel", , 1. Januar 2011 (2011-01-01), Seiten 1-1, XP55032730, Gefunden im Internet: URL:http://products.hospira.com/search/pro ductDetails?listNumber=DOCETAXEL_INJECTION &category=Drugs&page=Drugs&sort=ndc&order= asc [gefunden am 2012-07-13]
- "Use of a vial for sterilisation and sterile storage of a gel mass", , 1. Januar 2011 (2011-01-01), Seiten 1-1, XP55032735, Gefunden im Internet: URL:http://ais.badische-zeitung.de/piece/0 1/ee/01/ac/32375212.jpg [gefunden am 2012-07-13]
- Mark B Abelson: "Get to Know Your Antiseptic Options: The most efficient ways to eradicate microorganisms from the surgical field and the patient's eye.", Review of Ophthalmology, 16. Dezember 2008 (2008-12-16), Seiten 1-6, XP55032737, Gefunden im Internet: URL:http://www.revophth.com/content/d/ther apeutic_topics/i/1222/c/22999/ [gefunden am 2012-07-13]
- J. Wesley Alexander: "History of the Medical Use of Silver", SURGICAL INFECTIONS, vol. 10, no. 3, 1 June 2009 (2009-06-01), pages 289-292, XP55288836, US ISSN: 1096-2964, DOI: 10.1089/sur.2008.9941

## Beschreibung

Die vorliegende Erfindung betrifft ein Vorratsgefäß enthaltend mindestens einen im Inneren des Vorratsgefäßes angeordneten antibakteriell wirkenden Formkörper, der aus einer Formmasse gebildet ist, die mindestens eine Sorte eines partikulären antibakteriellen Materials enthält. Die Erfindung zeichnet sich dadurch aus, dass der Formkörper einen zylindrischen Grundkörper aufweist, wobei der Grundkörper spezielle Bemessungen aufweist, die vom mittleren Teilchendurchmesser des eingesetzten partikulären antibakteriellen Materials abhängen.

Die WO 2009/109370 A1 betrifft eine Dosierungsvorrichtung zur dosierten Abgabe von Flüssigpräparaten, insbesondere zur Dosierung von medizinischen, pharmazeutischen und kosmetischen Flüssigpräparaten, wobei auf die Verwendung von Konservierungsmitteln verzichtet werden kann.

In der EP 0 473 892 A2 wird eine Fluid-Abgabeeinrichtung für ein keimfreies Fluid, insbesondere für Augentropfen, beschrieben mit einem Vorratsbehälter, dessen Abgabekanal eine im Fluid lösbare Substanz mit oligodynamischer Wirkung enthält. Dabei ist die Abgabeeinrichtung als luftausgleichsfrei arbeitende Dosierpumpe mit einem die Einlassöffnung verschließbaren Einlassventil ausgebildet und die im Fluid lösbare oligodynamisch wirksame Substanz ist im Bereich des Einlassventils oder dessen Zu- und/oder Ablauf angeordnet.

Aus der EP 1 496 086 A1 ist eine antibakterielle Glaszusammensetzung bekannt, die ein antibakterielles Glas und einen anorganischen Füllstoff enthält, der in der antibakteriellen Glaszusammensetzung dispergiert ist. Diese Glaszusammensetzung kann beispielsweise als Pellets oder als Paste vorliegen, ebenso ist es möglich, die Zusammensetzung in Form eines Films, eines Formkörpers oder einer Beschichtungszusammensetzung bereitzustellen. Hieraus lassen sich beispielsweise Textilien oder Sheets herstellen.

Problematisch bei derartigen Formkörpern ist jedoch, dass die antibakterielle Wirkung einen relativ hohen Gehalt des antibakteriellen Glases erfordert, da die antibakterielle Wirkung darauf zurückzuführen ist, dass die antibakteriellen Glaspartikel an der Oberfläche der Zusammensetzung angeordnet sein müssen, um ihre antibakterielle Wirkung durch Kontakt mit den jeweiligen Bakterien zu entfalten.

Ausgehend von dieser Problematik ist es Aufgabe der vorliegenden Erfindung, ein Vorratsgefäß enthaltend einen antibakteriell wirkenden Formkörper anzugeben, der durch Wahl seiner Form bei gleicher Beladung mit einem antibakteriell wirkenden Material eine erhöhte antibakterielle Wirkung aufweist.

Diese Aufgabe wird durch das Vorratsgefäß mit den Merkmalen des Patentanspruchs 1 gelöst. Patentanspruch 12 gibt ein Verfahren zur sterilen Lagerung von Formulierungen mittels des erfindungsgemäßen Formkörpers an. Schließlich werden mit Patentanspruch 13 Verwendungszwecke des Vorratsgefäßes beschrieben. Die jeweiligen abhängigen Patentansprüche stellen dabei vorteilhafte Weiterbildungen dar.

Erfindungsgemäß wird somit ein Vorratsgefäß, enthaltend mindestens einen im Inneren des Vorratsgefäß angeordneten antibakteriell wirkenden Formkörper angegeben, der aus einer Formmasse gebildet ist, die mindestens eine Sorte eines partikulären antibakteriellen Materials mit einem mittleren Teilchendurchmesser, das in mindestens einem Kunstharz dispergiert vorliegt, umfasst, wobei dem Formkörper ein zylindrischer Grundkörper, dessen maximaler Durchmesser der Grundfläche höchstens das 100-fache des mittleren Teilchendurchmessers des partikulären antibakteriellen Materials beträgt und der zu einer Spirale aufgewickelt oder in Form eines wirrgelegten Knäuels vorliegt, zugrunde liegt.

Erfindungsgemäß enthält das Vorratsgefäß steril gelagerte Flüssigkeiten, Lösungen, Dispersionen, Suspensionen, Gele, Organogele, Mikroemulsionsgele, Pasten und/oder Cremes für pharmazeutische, kosmetische und/oder Lebensmittelanwendungen, wobei der Formkörper zur Sterilhaltung der Flüssigkeiten, Lösungen, Dispersionen, Suspensionen, Gele, Organogele, Mikroemulsionsgele, Pasten und/oder Cremes bei der Lagerung dient.

Das Prinzip, das der vorliegenden Erfindung zugrunde liegt, ist somit, dass der maximale Durchmesser des zylindrischen Grundkörpers in Relation zum mittleren Teilchendurchmesser des partikulären antibakteriellen Materials bestimmt ist.

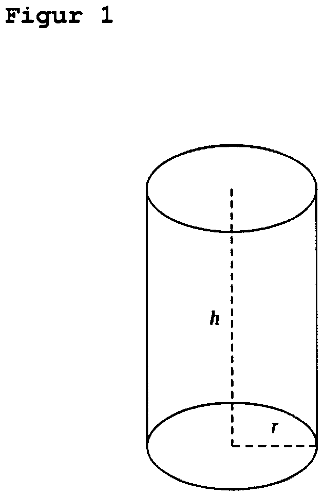

Ein beispielhafter zylindrischer Grundkörper ist dabei in Figur 1 dargestellt. Figur 1 zeigt einen Zylinder mit kreisförmiger Grundfläche, die einen Radius r aufweist. Die Höhe bzw. Länge des Zylinders ist dabei mit h bemessen. Der Durchmesser der Grundfläche des Zylinders (d.h. des Kreises) beträgt somit 2r. Erfindungswesentlich ist dabei nun, dass der Durchmesser der Grundfläche, d.h. 2r, so bemessen ist, dass maximal das 100-fache des mittleren Teilchendurchmessers des partikulären antibakteriellen Materials gegeben ist.

Dieser Zylinder mit kreisförmiger Grundfläche stellt jedoch nur eine mögliche Ausführungsform eines zylindrischen Grundkörpers dar. Ebenso kann die Möglichkeit gegeben sein, dass die Grundfläche des zylindrischen Grundkörpers elliptisch, n-eckig, mit 3 ≤ n ≤ 10, oder unregelmäßig ist. Hierzu zählen beispielsweise auch sternförmige Grundflächen.

Das Prinzip der vorliegenden Erfindung beruht somit darauf, dass ein bestimmter Durchmesser eines Grundkörpers kleiner bemessen ist als das 100-fache des mittleren Teilchendurchmessers des partikulären antibakteriellen Materials, das in der Formmasse, aus der der Grundkörper besteht, eingebettet ist. Dies bewirkt, dass lediglich ein geringer Teil des partikulären antibakteriellen Materials vollständig von der Formmasse umschlossen ist und ein relativ großer Anteil dieses Materials so im Formkörper angeordnet ist, dass zumindest ein Teil der Oberfläche des partikulären antibakteriellen Materials aus dem Formkörper herausragt, d.h. auf der Oberfläche des Formkörpers angeordnet ist. Diese Teilchen aus antibakteriellem Material sind somit nicht vollständig von der Formmasse, die den antibakteriell wirkenden Formkörper bildet, umschlossen, so dass, bezogen auf den Gesamtanteil des partikulären antibakteriellen Materials, ein relativ großer Anteil auf der Oberfläche des Formkörpers angeordnet ist und somit antibakterielle Wirkung entfalten kann. Durch diesen relativ hohen Anteil des an der Oberfläche des Grundkörpers angeordneten antibakteriellen Materials kann der Formkörper, verglichen mit anderen Formmassen, bei denen ein höherer Anteil des antibakteriellen Materials vollständig von der Formmasse umschlossen ist, eine relativ hohe antibakterielle Wirkung entfalten.

Ebenso bringt der Ausdruck "Grundkörper" zum Ausdruck, dass es sich hierbei nicht um einen starren oder geometrisch festgelegten Grundkörper handeln muss, der den idealen geometrischen zugrunde liegenden Prinzipien, wie beispielsweise Zylinder, genügt. In Figur 1 ist beispielsweise ein idealer Zylinder dargestellt, der sich entlang einer geraden Achse, die mit h bezeichnet ist, erstreckt. Der Begriff des Grundkörpers gemäß der vorliegenden Erfindung umfasst ebenso Ausführungsformen, bei denen die Längsachse eines derartigen Zylinders nicht gerade, sondern beispielsweise geschwungen oder gebogen verläuft. Dies gilt selbstverständlich ebenso für andere Zylinderformen, wie z.B. den Quader als spezielle Form des Zylinders.

Für den Grundkörper, der als Zylinder vorliegt, kann am besten ein Vergleich mit einem drahtförmigen Grundkörper angestellt werden. Für den Fall, dass der Zylinder einen geradlinigen Verlauf hat, liegt ein gerader Draht vor, der jedoch ebenso gebogen vorliegen kann.

Die vorliegende Erfindung sieht vor, dass von einem zylindrischen Grundkörper ausgegangen wird und dieser zu einer Spirale aufgewickelt oder in Form eines wirrgelegten Knäuels vorliegt. Diese Ausführungsform bewirkt, dass eine kompaktere Ausgestaltung des erfindungsgemäßen Formkörpers vorliegt, so dass die antibakterielle Wirkung auf kleinste Raumvolumina konzentriert werden kann.

Eine besonders bevorzugte Ausführungsform sieht vor, dass der maximale Durchmesser der Grundfläche des zylinderförmigen Grundkörpers vom 0,5 bis 80-fachen, bevorzugt vom 1 bis 60-fachen, besonders bevorzugt vom 2 bis 30-fachen, des mittleren Teilchendurchmessers des partikulären antibakteriellen Materials beträgt.

Für den zylindrischen Grundkörper ergeben sich die folgenden bevorzugten absoluten Bemaßungen:
Die Länge bzw. Höhe des zylindrischen Formkörpers beträgt bevorzugt von 1 mm bis 100 cm, weiter bevorzugt von 5 mm bis 80 cm, besonders bevorzugt von 10 mm bis 60 cm und/oder der maximale Durchmesser der Grundfläche des zylindrischen Grundkörpers ist kleiner 5 mm, bevorzugt von 0,01 bis 0,9 mm.

In einer weiteren bevorzugten Ausführungsform ist die mindestens eine Sorte des partikulären antibakteriellen Materials ein partikuläres antibakterielles Glas, insbesondere ein partikuläres antibakterielles Glas ausgewählt aus der Gruppe bestehend aus Elementglas, Oxidglas, Silicatglas, Silicaglas, Alkalisilicatglas, Kalknatronglas, Blei(alkali)glas, Bariumglas, Borsilicatglas, Phosphatglas, Boratglas, Fluoridglas, Chloridglas, Sulfidglas, Carbonatglas, Nitratglas, Sulfatglas, wasserlöslichem Glas, kristallisiertem Glas sowie Mischungen hieraus.

Bevorzugt ist die Oberfläche der Partikel der mindestens einen Sorte des partikulären antibakteriellen Materials zumindest teilweise oder ganz mit einem antibakteriell wirkenden Metall, insbesondere einem Metall ausgewählt aus der Gruppe bestehend aus Silber, Kupfer, Zink und/oder Mischungen oder Kombinationen hieraus, beschichtet und/oder enthält Ionen und/oder Kolloide der zuvor genannten Metalle.

Ferner ist vorteilhaft, wenn der mittlere Partikeldurchmesser der Partikel des partikulären antibakteriellen Materials 0,05 bis 50 µm, bevorzugt 0,1 bis 25 µm, besonders bevorzugt 2 bis 10 µm, beträgt.

Bevorzugte Gehalte des Metalls und/oder der Metallionen betragen dabei, bezogen auf die Gesamtmasse an partikulärem antibakteriellen Material, von 0,01 Gew.-% oder mehr, bevorzugt von 0,01 bis 50 Gew.-%.

Zusätzlich kann die Formmasse mindestens einen partikulären anorganischen Füllstoff enthalten, der bevorzugt ausgewählt ist aus der Gruppe bestehend aus Barium und/oder Bariumsalzen, Silber oder Silbersalzen, Silica, Zeolithen, Kaolin, Talk, Zinkoxid sowie Mischungen hieraus.

Diese gegebenenfalls zusätzlich enthaltenen partikulären anorganischen Füllstoffe weisen dabei bevorzugte mittlere Teilchendurchmesser von 0,1 bis 10 µm auf.

Bevorzugte Kunstharze, die dem erfindungsgemäßen Formkörper zugrunde liegen können, sind dabei ausgewählt aus der Gruppe bestehend aus
a) Thermoplasten, insbesondere Polyvinylchlorid, Polyethylen, Polypropylen, Acrylnitril-Butadien-Styrol-Harzen (ABS-Harze), Acrylnitril-StyrolHarzen (AS-Harze), Polystyrol, Polyestern, Polyvinylidenchlorid, Polyamiden, Polybutylenterephthalat (PBT), gesättigten Polyestern, Polyacetalen, Polyvinylalkoholen, Polycarbonaten, Urethanharzen, Poly(meth)acrylaten, Polyacrylsäure, (teil)fluorierten Harzen, Ethylenvinylalkoholen (EVA) und/oder Kombinationen hieraus,
b) wärmehärtenden Harzen, insbesondere Siliconharzen, modifizierten Siliconharzen, ungesättigten Polyestern, Vinylesterharzen, Phenolharzen, Melaminharzen, Harnstoffharzen, Epoxidharzen und/oder Kombinationen hieraus,
c) Gummimaterialien, insbesondere auf Basis von natürlichem oder synthetischem Kautschuk und/oder Kombinationen hieraus, sowie
d) Mischungen aus den zuvor genannten Harzen.

Bevorzugte Gehalte der mindestens einen Sorte des partikulären antibakteriellen Materials betragen dabei, bezogen auf die Formmasse, mindestens 0,01 Gew.-%, bevorzugt 0,01 Gew.-% bis 50 Gew.-% und besonders bevorzugt 0,1 Gew.-% bis 15 Gew.-%.

Weiterhin ist es möglich, für den Fall, dass weiterhin partikuläre anorganische Füllstoffe eingearbeitet sind, dass diese einen Gehalt von 3 bis 50 Gew.-%, bezogen auf die mindestens eine Sorte des partikulären antibakteriellen Materials, enthalten können.

Erfindungsgemäß wird ebenso ein Verfahren zur sterilen Lagerung von Formulierungen ausgewählt aus der Gruppe bestehend aus Flüssigkeiten, Lösungen, Dispersionen, Suspensionen, Gelen, Organogelen, Mikroemulsionsgelen, Pasten und/oder Cremes, für pharmazeutische, kosmetische und/oder Lebensmittelanwendungen, angegeben, bei dem mindestens ein erfindungsgemäßer Formkörper mit den zuvor genannten Formulierungen in Kontakt gebracht wird.

Zudem betrifft die vorliegende Erfindung ein Vorratsgefäß, bei dem mindestens ein erfindungsgemäßer Formkörper im Inneren des Vorratsgefäßes angeordnet ist. Das Vorratsgefäß eignet sich dabei zur sterilen Lagerung von Flüssigkeiten, Lösungen, Dispersionen, Suspensionen, Gelen, Organogelen, Mikroemulsionsgelen, Pasten und/oder Cremes, für pharmazeutische, kosmetische und/oder Lebensmittelanwendungen, bzw. kann hierfür verwendet werden. Die zuvor genannten Komponenten, d.h. Flüssigkeiten, Lösungen, Dispersionen, Suspensionen, Gele, Organogele, Mikroemulsionsgele, Pasten und/oder Cremes, können beispielsweise vor Einbringen in das Vorratsgefäß und Lagerung dieser Komponenten mittels einem aus dem Stand der Technik bekannten Verfahren sterilisiert werden. Der Formkörper dient dann zur Sterilhaltung bei der Lagerung.

Ebenso ist es jedoch möglich und denkbar, dass die zuvor genannten Komponenten unsterisiliert in das Vorratsgefäß eingebracht werden, wobei bei erstem Kontakt mit dem erfindungsgemäßen antibakteriell wirkenden Formkörper eine Sterisilierung erfolgt. Bei beiden Varianten wird während des gesamten Zeitraums der Lagerung eine Sterilhaltung gewährleistet. Die vorliegende Erfindung wird anhand der nachfolgenden Figuren näher beschrieben.
Figur 1 zeigt einen kreisförmigen Zylinder als mögliche Grundform des erfindungsgemäßen Formkörpers.

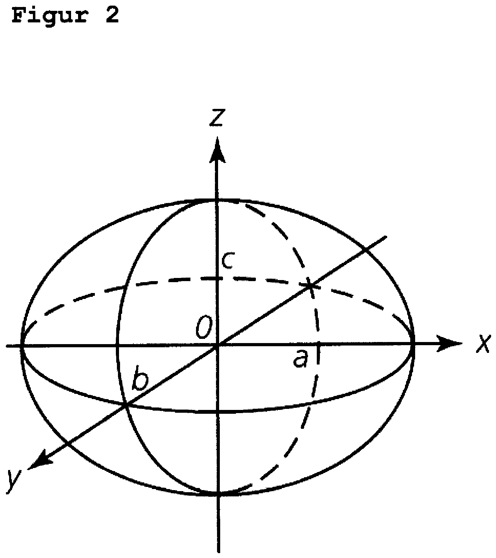

Figur 2 zeigt ein Ellipsoid als mögliche Grundform eines alternativen Formkörpers.

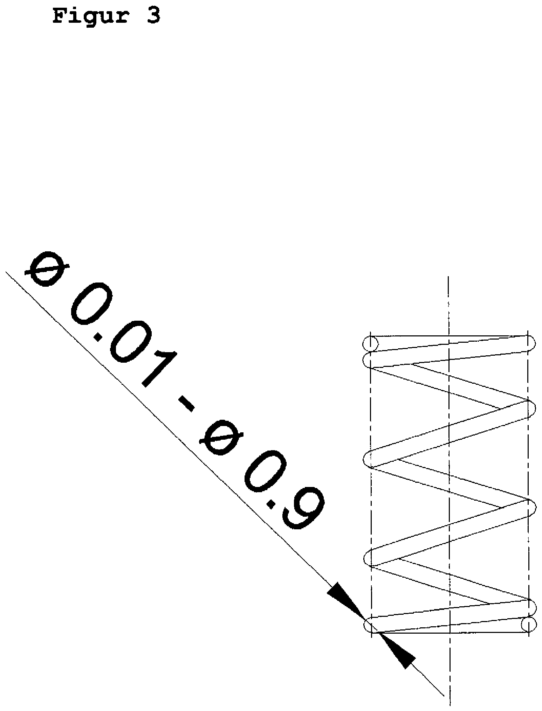

Figur 3 zeigt eine weitere Variante einer Ausführungsform des erfindungsgemäßen Formkörpers. Dieser weist dabei einen kreisförmigen zylindrischen Grundkörper auf, der zu einer Spirale mit ca. 4,5 Windungen aufgewickelt vorliegt. Der bevorzugte Durchmesser der Grundfläche des Grundkörpers, d.h. der kreisförmigen Grundfläche des Zylinders, beträgt dabei bevorzugt von 0,01 bis 0,9 mm.

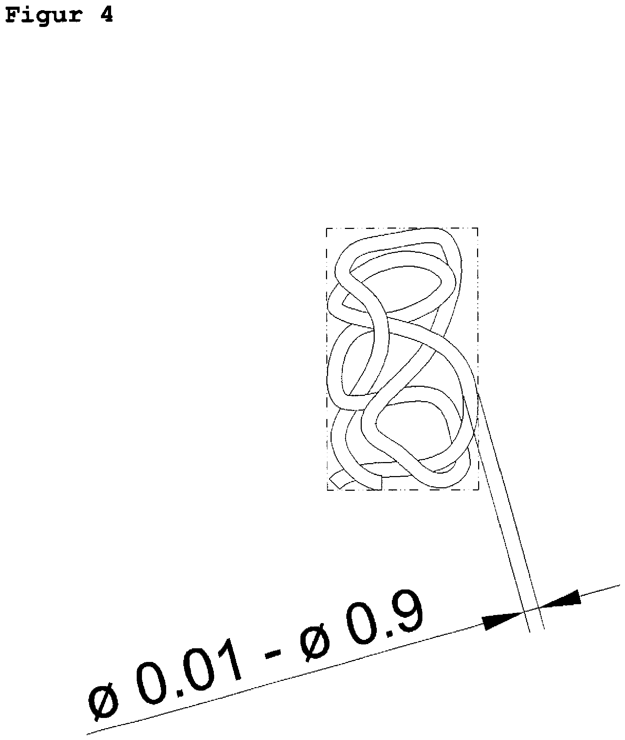

Figur 4 betrifft eine weitere bevorzugte geometrische Ausführungsform eines erfindungsgemäßen Grundkörpers, der in diesem Fall als wirrgelegtes Knäuel vorliegt. Auch diesem Formkörper liegt ein kreisförmiger zylindrischer Grundkörper zugrunde, der durch entsprechende Aufwicklung zum wirrgelegten Knäuel gefertigt werden kann. Der Durchmesser der kreisförmigen Grundfläche des Zylinders beträgt auch hier bevorzugt von 0,01 bis 0,9 mm.

## Patentansprüche

1. Vorratsgefäß, enthaltend mindestens einen im Inneren des Vorratsgefäßes angeordneten antibakteriell wirkenden Formkörper, wobei der antibakteriell wirkende Formkörper aus einer Formmasse, umfassend mindestens eine Sorte eines partikulären antibakteriellen Materials mit einem mittleren Teilchendurchmesser, das in mindestens einem Kunstharz dispergiert vorliegt, gebildet ist,
**dadurch gekennzeichnet,**
**dass** dem Formkörper
ein zylindrischer Grundkörper, dessen maximaler Durchmesser der Grundfläche höchstens das 100-fache des mittleren Teilchendurchmessers des partikulären antibakteriellen Materials beträgt und der zu einer Spirale aufgewickelt oder in Form eines wirrgelegten Knäuels vorliegt,
zugrunde liegt,
wobei das Vorratsgefäß steril gelagerte Flüssigkeiten, Lösungen, Dispersionen, Suspensionen, Gele, Organogele, Mikroemulsionsgele, Pasten und/oder Cremes für pharmazeutische, kosmetische und/oder Lebensmittelanwendungen enthält, und
wobei der Formkörper zur Sterilhaltung der Flüssigkeiten, Lösungen, Dispersionen, Suspensionen, Gelen, Organogelen, Mikroemulsionsgelen, Pasten und/oder Cremes bei der Lagerung dient.

2. Vorratsgefäß nach Anspruch 1, **dadurch gekennzeichnet, dass** der maximale Durchmesser der Grundfläche des zylinderförmigen Grundkörpers vom 0,5 bis 80-fachen des mittleren Teilchendurchmessers des partikulären antibakteriellen Materials beträgt.

3. Vorratsgefäß nach einem der vorhergehenden Ansprüche, **dadurch gekennzeichnet, dass**
a) die Länge des zylindrischen Formkörpers von 1 mm bis 100 cm und/oder
b) der maximale Durchmesser der Grundfläche des zylindrischen Grundkörpers kleiner 5 mm
beträgt.

4. Vorratsgefäß nach einem der vorhergehenden Ansprüche, **dadurch gekennzeichnet, dass** die Grundfläche des zylindrischen Grundkörpers kreisförmig, elliptisch, n-eckig, mit 3 ≤ n ≤ 10, oder unregelmäßig ist.

5. Vorratsgefäß nach einem der vorhergehenden Ansprüche, **dadurch gekennzeichnet, dass** die mindestens eine Sorte des partikulären antibakteriellen Materials ein partikuläres antibakterielles Glas ist.

6. Vorratsgefäß nach einem der vorhergehenden Ansprüche, **dadurch gekennzeichnet, dass** die Oberfläche der Partikel der mindestens einen Sorte des partikulären antibakteriellen Materials zumindest teilweise oder ganz mit einem antibakteriell wirkenden Metall beschichtet ist und/oder Ionen und/oder Kolloide der zuvor genannten Metalle enthält.

7. Vorratsgefäß nach vorhergehendem Anspruch, **dadurch gekennzeichnet, dass**
a) der mittlere Partikeldurchmesser der Partikel des partikulären antibakteriellen Materials 0,05 bis 50 µm und/oder
b) der Gehalt des Metalls und/oder der Metallionen, bezogen auf die Gesamtmasse an partikulärem antibakteriellen Material von 0,01 Gew.-% oder mehr
beträgt.

8. Vorratsgefäß nach einem der vorhergehenden Ansprüche, **dadurch gekennzeichnet, dass** die Formmasse zusätzlich mindestens einen partikulären anorganischen Füllstoff enthält.

9. Vorratsgefäß nach vorhergehendem Anspruch, **dadurch gekennzeichnet, dass** der mindestens eine partikuläre Füllstoff einen mittleren Teilchendurchmesser von 0,1 bis 10 µm aufweist.

10. Vorratsgefäß nach einem der vorhergehenden Ansprüche, **dadurch gekennzeichnet, dass** das mindestens eine Kunstharz ausgewählt ist aus der Gruppe bestehend aus
a) Thermoplasten,
b) wärmehärtenden Harzen,
c) Gummimaterialien, sowie
d) Mischungen aus den zuvor genannten Harzen.

11. Vorratsgefäß nach einem der vorhergehenden Ansprüche, **dadurch gekennzeichnet, dass** der Gehalt
a) der mindestens einen Sorte des partikulären antibakteriellen Materials, bezogen auf die Formmasse, mindestens 0,01 Gew.-% und/oder
b) ggf. des mindestens einen partikulären anorganischen Füllstoffs, bezogen auf die mindestens eine Sorte des partikulären antibakteriellen Materials, 3 bis 50 Gew.-%
beträgt.

12. Verfahren zur sterilen Lagerung von Formulierungen ausgewählt aus der Gruppe bestehend aus Flüssigkeiten, Lösungen, Dispersionen, Suspensionen, Gelen, Organogelen, Mikroemulsionsgelen, Pasten und/oder Cremes für pharmazeutische, kosmetische und/oder Lebensmittelanwendungen bei dem mindestens ein antibakteriell wirkender Formkörper mit den zuvor genannten Formulierungen in Kontakt gebracht wird, wobei der antibakteriell wirkende Formkörper aus einer Formmasse, umfassend mindestens eine Sorte eines partikulären antibakteriellen Materials mit einem mittleren Teilchendurchmesser, das in mindestens einem Kunstharz dispergiert vorliegt, gebildet ist, wobei dem Formkörper
ein zylindrischer Grundkörper, dessen maximaler Durchmesser der Grundfläche höchstens das 100-fache des mittleren Teilchendurchmessers des partikulären antibakteriellen Materials beträgt und der zu einer Spirale aufgewickelt oder in Form eines wirrgelegten Knäuels vorliegt,
zugrunde liegt.

13. Verwendung eines Vorratsgefäßes gemäß einem der Ansprüche 1 bis 11 zur Sterilisierung und/oder zur sterilen Lagerung von Flüssigkeiten, Lösungen, Dispersionen, Suspensionen, Gelen, Organogelen, Mikroemulsionsgelen, Pasten und/oder Cremes für pharmazeutische, kosmetische und/oder Lebensmittelanwendungen.

## Claims

1. Storage vessel, comprising at least one antibacterially-acting moulded article, disposed in the interior of the storage vessel, wherein the antibacterially-acting moulded article is formed from a moulding compound, comprising at least one sort of a particular antibacterial material with an average particle diameter which is present dispersed in at least one synthetic resin,
***characterised in that***
the basis of the moulded article is
an cylindrical basic body, the maximum diameter of the base of which is at most 100 times the average particle diameter of the particular antibacterial material and which is coiled to form a spiral or is present in the form of a randomly orientated ball,
wherein the storage vessel contains sterile stored liquids, solutions, dispersions, suspensions, gels, organogels, microemulsion gels, pastes and/or creams for pharmaceutical, cosmetic and/or foodstuff applications, and
wherein the moulded article serves for maintaining sterility of the liquids, solutions, dispersions, suspensions, gels, organogels, microemulsion gels, pastes and/or creams during the storage.

2. Storage vessel according to claim 1, **characterised in that** the maximum diameter of the base of the cylindrical basic body is from 0.5 to 80 times the average particle diameter of the particular antibacterial material.

3. Storage vessel according to one of the preceding claims, **characterised in that**
a) the length of the cylindrical moulded article is from 1 mm to 100 cm and/or
b) the maximum diameter of the base of the cylindrical basic body is less than 5 mm.

4. Storage vessel according to one of the preceding claims, **characterised in that** the base of the cylindrical basic body is circular, elliptical, n-angular, with 3 ≤ n ≤ 10, or is irregular.

5. Storage vessel according to one of the preceding claims, **characterised in that** the at least one sort of particular antibacterial material is a particular antibacterial glass.

6. Storage vessel according to one of the preceding claims, **characterised in that** the surface of the particles of the at least one sort of particular antibacterial material is coated at least partially or entirely with an antibacterially-acting metal and/or comprises ions and/or colloids of the previously mentioned metals.

7. Storage vessel according to the preceding claim, **characterised in that**
a) the average particle diameter of the particles of the particular antibacterial material is 0.05 to 50 µm and/or
b) the content of the metal and/or of the metal ions, relative to the total mass of particular antibacterial material, is of 0.01% by weight or more.

8. Storage vessel according to one of the preceding claims, **characterised in that** the moulding compound comprises in addition at least one particular inorganic filler.

9. Storage vessel according to the preceding claim, **characterised in that** the at least one particular filler has an average particle diameter of 0.1 to 10 µm.

10. Storage vessel according to one of the preceding claims, **characterised in that** the at least one synthetic resin is selected from the group consisting of
a) thermoplastics,
b) heat-curing resins,
c) rubber materials, and also
d) mixtures of the previously mentioned resins.

11. Storage vessel according to one of the preceding claims, **characterised in that** the content
a) of the at least one sort of particular antibacterial material, relative to the moulding compound, is at least 0.01% by weight and/or
b) if necessary of the at least one particular inorganic filler, relative to the at least one sort of particular antibacterial material, is 3 to 50% by weight.

12. Method for sterile storage of formulations selected from the group consisting of liquids, solutions, dispersions, suspensions, gels, organogels, microemulsion gels, pastes and/or creams for pharmaceutical, cosmetic and/or foodstuff applications, in which at least one antibacterially-acting moulded article is brought in contact with the previously mentioned formulations, wherein the antibacterially-acting moulded article is formed from a moulding compound, comprising at least one sort of a particular antibacterial material with an average particle diameter which is present dispersed in at least one synthetic resin,
wherein the moulding article is based on
an cylindrical basic body, the maximum diameter of the base of which is at most 100 times the average particle diameter of the particular antibacterial material and which is coiled to form a spiral or is present in the form of a randomly orientated ball.

13. Use of a storage vessel according to one of the claims 1 to 11 for sterilisation and/or for sterile storage of liquids, solutions, dispersions, suspensions, gels, organogels, microemulsion gels, pastes and/or creams for pharmaceutical, cosmetic and/or foodstuff applications.

## Revendications

1. Réservoir, contenant au moins un corps moulé antibactérien disposé à l'intérieur du réservoir, le corps moulé antibactérien étant constitué d'une masse moulée, comprenant au moins une sorte de matériau antibactérien particulaire, avec un diamètre de particules moyen, qui est dispersé dans au moins une résine synthétique,
**caractérisé en ce que**
le corps moulé est basé sur un corps de base cylindrique dont le diamètre maximal de la surface de base représente au maximum 100 fois le diamètre moyen des particules du matériau antibactérien particulaire et est enroulé en spirale ou existe sous la forme d'une pelote emmêlée,
le réservoir contenant des liquides, des solutions, des dispersions, des suspensions, des gels, des gels organiques, des gels à micro-émulsions, des pâtes et/ou des crèmes pour des applications pharmaceutiques, cosmétiques et/ou alimentaires stockés de manière stérile et
le corps moulé servant à conserver de manière stérile les liquides, solutions, dispersions, suspensions, gels, gels organiques, gels à micro-émulsions, pâtes et/ou crèmes lors du stockage.

2. Réservoir selon la revendication 1, **caractérisé en ce que** le diamètre maximal de la surface de base du corps de base cylindrique représente de 0,5 à 80 fois le diamètre moyen des particules du matériau antibactérien particulaire.

3. Réservoir selon l'une des revendications précédentes, **caractérisé en ce que**
a) la longueur du corps moulé cylindrique est de 1 mm à 100 cm et/ou
b) le diamètre maximal de la surface de base du corps de base cylindrique est inférieur à 5 mm.

4. Réservoir selon l'une des revendications précédentes, **caractérisé en ce que** la surface de base du corps de base cylindrique est circulaire, elliptique, n-angulaire, avec 3 ≤ n ≤ 10 ou irrégulière.

5. Réservoir selon l'une des revendications précédentes, **caractérisé en ce que** l'au moins une sorte de matériau antibactérien particulaire est un verre antibactérien particulaire.

6. Réservoir selon l'une des revendications précédentes, **caractérisé en ce que** la surface des particules de l'au moins une sorte de matériau antibactérien particulaire est revêtue au moins partiellement ou entièrement d'un métal et/ou d'ions et/ou de colloïdes des métaux à action antibactérienne mentionnés ci-dessus.

7. Réservoir selon la revendication précédente, **caractérisé en ce que**
a) le diamètre moyen des particules du matériau antibactérien particulaire est de 0,05 à 50 µm et/ou
b) la teneur en métal et/ou en ions métalliques, par rapport à la masse totale de matériau antibactérien particulaire est de 0,01 % en poids ou plus.

8. Réservoir selon l'une des revendications précédentes, **caractérisé en ce que** la masse moulée contient en outre au moins une charge anorganique particulaire.

9. Réservoir selon la revendication précédente, **caractérisé en ce que** l'au moins une charge particulaire présente une taille moyenne de particules de 0,1 à 10 µm.

10. Réservoir selon la revendication précédente, **caractérisé en ce que** l'au moins une résine synthétique est sélectionnée dans le groupe constitué de
a) thermoplastiques,
b) résines thermodurcissables,
c) matériaux de type caoutchouc et
d) mélanges des résines mentionnées ci-dessus.

11. Réservoir selon la revendication précédente, **caractérisé en ce que** la teneur
a) de l'au moins une sorte de matériau antibactérien particulaire, par rapport à la masse moulée, est d'au moins 0,01 % en poids et/ou
b) le cas échéant de l'au moins une charge anorganique particulaire, par rapport à l'au moins une sorte de matériau antibactérien particulaire est de 3 à 50 % en poids.

12. Procédé de stockage stérile de formulations sélectionnées dans le groupe constitué de liquides, de solutions, des dispersions, de suspensions, de gels, de gels organiques, de micro-émulsions, de pâtes et/ou de crèmes pour des applications pharmaceutiques, cosmétiques et/ou alimentaires, dans lequel au moins un corps moulé antibactérien est mise en contact avec les formulations mentionnées ci-dessus, le corps moulé antibactérien étant constitué d'une masse moulée comprenant au moins une sorte de matériau antibactérien particulaire avec une taille de particules moyenne, qui est dispersé dans au moins une résine,
le corps moulé étant basé sur un corps de base cylindrique dont le diamètre maximal de la surface de base représente au maximum 100 fois le diamètre moyen des particules du matériau antibactérien particulaire et est enroulé en spirale ou existe sous la forme d'une pelote emmêlée.

13. Utilisation d'un réservoir selon l'une des revendications 1 à 11 pour la stérilisation et/ou le stockage stérile de liquides, de solutions, des dispersions, de suspensions, de gels, de gels organiques, de micro-émulsions, de pâtes et/ou de crèmes pour des applications pharmaceutiques, cosmétiques et/ou alimentaires.
